# EUROPEAN PATENT APPLICATION

(11) **EP 2 241 198 A1**
(43) Date of publication of application: **20.10.2010**
(21) Application number: 10159281.4
(22) Date of filing: 07.04.2010
(51) Int. Cl.: A23L 1/30, A23L 1/305, A61K 31/195, A61K 36/00

(54) **Mucositis prevention supplement and treatment**

(30) Priority: 07.04.2009 EP 09005111
(71) Applicant: Fresenius Kabi Deutschland GmbH, 61352 Bad Homburg (DE)
(72) Inventor: Schneid, Christina, 55118, Mainz (DE)
(74) Representative: Richly, Erik

(57) **Abstract**

The present invention provides for a nutritional composition comprising from 0.25 g to 25 g of glutamine, in neutral or salt form or precursor or derivative thereof; and from 0.1 g to 10 g of a phytochemical or a mixture thereof, wherein the phytochemical is an antioxidant. The nutritional composition is particularly useful in the treatment and/or prevention of mucositis.

## Description

### Technical Field

The present invention relates to a nutritional composition, preferably a nutritional supplement, for the prevention and/or treatment of mucositis. The composition according to the present invention contains as constituents glutamine, both in neutral or salt form or a precursor or derivative thereof and at least one phytochemical. In a preferred embodiment, the composition additionally contains one or more further antioxidants such as carotenoids, vitamin E, vitamin C, zinc and selenium. In a particular preferred embodiment the composition additional has a product consistency made for cooling and suckling.

### Background Art

Oral and intestinal mucositis, further summarized as mucositis, is a painful inflammation and ulceration of the mucous membranes lining the digestive tract and is a common complication of cytoreductive cancer chemotherapy and radiotherapy. Mucositis is typically associated with pain, increased risk of infections, impaired nutritional status and inadequate hydration as well as increased risk of poorer outcome due to treatment interruptions.

Oral mucositis is the dose-limiting toxicity of treatment modalities like accelerated fractionated and hyperfractionated radiotherapy and of interventions that combine chemotherapy and radiotherapy. Its counterpart, gastrointestinal (GI) mucositis, is a well recognized toxicity associated with some standard-dose chemotherapy regimens commonly used in cancer treatment and with radiotherapy encompassing any area of the GI tract. Mucositis is an especially severe problem for patients who are undergoing hematopoietic stem-cell transplantation (HSCT) because of the high-dose, myeloablative chemotherapy used for conditioning. In the transplant setting, oral mucositis is often so severe that patients require intravenous narcotics for relief of pain and, if oral intake is impossible, total parenteral nutrition. Oral mucositis may occur in up to 100 % of patients undergoing high-dose chemotherapy with HSCT. For patients receiving this treatment, a 1-point increase in an oral mucositis score has been found to be associated with a significant increase in days with fever, risk of infection, additional days of parenteral nutrition, use of intravenous narcotic analgesics, total hospital charges, and 100-day mortality. From the patient's perspective, oral mucositis is often the single most debilitating side effect of transplantation. Recognizing the dramatic clinical and psychological effects of oral mucositis and the barrier that this condition sometimes becomes tremendously severe, that to some extent life-saving therapy could not be followed.

From a patient's viewpoint mucositis is one of the most troublesome side effects of cancer treatments occurring in almost 50% of patients receiving high-dose chemotherapy and in up to 80% of patients with head and neck cancer treated with radiotherapy. Mucositis significantly decreases the quality of life, incurs great economic costs, and interrupts treatment cycles.

Radiotherapy associated mucositis usually appears at the end of the second week of treatment lasting for six to eight weeks. As a result of cell death in reaction to chemo- or radiotherapy, the mucosal lining of the mouth becomes thin, may slow off and then become red, inflamed and ulcerated. The ulcers may become covered by a yellowish white fibrin clot called a pseudomembrane. Ulcers may range from 0.5 cm to greater than 4 cm and can cause severe pain. The degree of pain is usually related to the extent of the tissue damage. Patients may experience trouble in speaking, eating, swallowing disorders, or even opening the mouth due to the pain. Patients receiving concomitant radiation therapy to the neck and mouth area may experience an alteration in taste perception because of effects on taste spots that are mostly located in the tongue.

The diagnosis is based on the symptoms the patient is experiencing and the appearance of the tissues of the mouth following chemotherapy, bone marrow transplants or radiotherapy.

For the time being there is no established therapy for the nutritional or pharmaco-nutritional intervention. The idea is to filter out patients with a high risk of oral mucositis development and to treat them already from the beginning of anti-cancer therapy on with an adopted pharmaco-nutritional approach.

Treatment and/or prevention of mucositis is mainly supportive. Oral hygiene, the administration of a plenty of liquids and the avoidance of alcohol, citrus fruits and hot foods are the common recommendations for treating mucositis. Experimental therapies concerning the use of amino acid supplementation or vitamins have been reported. US 6,479,068 B describes a daily regimen for oncology patients suffering from mucositis, stomatis and cachexia wherein the daily regimen involves the administration of a composition comprising L-glutamine, vitamins and selenium. US 2003/0082249 Adescribes a composition for use in treating or preventing mucositis and/or xerostomia comprising capsaicin or capsaicin derivatives and other additional compounds.

However, the compositions of the prior art still fail to provide for a satisfactory treatment and/or prevention of mucositis. In particular, the ideal key nutrient composition of preventive intervention of mucositis has still not been determined.

### Disclosure of Invention

Therefore, the object of the present invention is to provide for nutritional compositions which are highly effective in the prevention and/or treatment of mucositis.

The present invention provides for a nutritional composition comprising from 0.25 g to 25 g of glutamine, in neutral or salt form or precursor or derivative thereof; and from 0.1 g to 10 g of a phytochemical or a mixture thereof, wherein the phytochemical is an antioxidant. It has been found by the inventors that a combination of glutamine and a phytochemical according to the invention surprisingly acts in a synergistic manner and is more efficient in the treatment and/or prevention of mucositis than the two components alone. Particularly, an improved healing of the mucosa in mucositis and/or a markedly decreased frequency of mucositis following radio- or chemotherapy can be achieved using the compositions according to the invention. In another embodiment the composition additionally comprises one or more antioxidants selected from the group consisting of carotenoids, vitamin E, vitamin C, zinc and selenium. In particular embodiments the amount of glutamine, of the phytochemical and/or of the carotenoids, vitamin E, vitamin C, zinc and selenium is based on 100 kcal of the total composition

A first component of the composition according to the present invention is glutamine, which belongs to the group of semi-essential amino acids. Glutamine may be comprised in neutral or salt form or in the form of a precursor or a derivative thereof. Without being bound by any theory, glutamine is believed to be involved in the regulation of mucosa protein synthesis and degradation. Glutamine is a protector of the bowel, i.e. protecting from radiation induced mucosal injury.

Significant glutamine depletion occurs in patients with cancer, especially those receiving chemotherapy or radiotherapy. The extent of tissue damage may depend on adequate stores of glutamine in tissues, especially those of the gastrointestinal tract. Protective effects of glutamine supplementation according to the invention prevent toxicities associated with chemotherapy and radiotherapy. Glutamine is also a very important substrate for macrophages, supporting the immune-system and favouring anti-inflammatory cytokines which counteract the inflammatory status.

The composition of the invention comprises from 0.25 g to 25 g glutamine, preferably from 1 to 25 g, from 2.5 to 18 g, ≥ 5 g and ≤ 18 g, ≥ 10 g and ≤ 18 g, ≥ 2.5 g and ≤ 10 g, or ≥ 3 g and ≤ 7 g glutamine. Most preferably the composition comprises ≥ 10 g and ≤ 15 g or ≥ 4 g and ≤ 5 g of glutamine.

In one embodiment of the invention the amount of glutamine stated in the preceding paragraph is based on 100 kcal of the total composition.

In another embodiment of the invention the amounts of glutamine given above are comprised by a single dose of the composition of the invention to be administered to a patient. The term "single dose" refers to the amount of composition according to the invention that is administered to the patient in a continuous manner, i.e. without larger interruptions. Thus, a "single dose" may refer to one dose that is administered to the patient or it may refer to two, three, four or more sub-doses that are administered to the patient without larger interruptions of more than 10, 20, 30 or 60 minutes between two consecutive sub-doses. As an example, a "single dose" may consist of two or three sub-doses which are consecutively administered to the patient shortly before and during radiotherapy. If more than one single dose is administered per day the single doses can be of identical or different glutamine content.

When the amount of glutamine in the composition is within the above mentioned ranges, the anti-mucositis effect of the composition is excellent.

As a second essential component the composition according to the composition of the present invention comprises at least one phytochemical or a mixture of more than one phytochemical, wherein the phytochemical provides an anti-oxidative capacity, i.e. acts as an antioxidant. Antioxidants slow down or prevent the oxidation of other molecules and/or terminate chain reactions caused by free radicals.

In a preferred embodiment the phytochemical further has an anti-inflammatory effect.

The term "phytochemical" according to the present invention should be understood to comprise any plant extract comprising compounds found in plants that have a beneficial effect on health or an active role in the amelioration of a disease, but that are not required for a normal functioning of the body. Phytochemicals are also referred to as phytonutrients. However, according to the present invention the term "phytochemicals" or "phytonutrient" or "secondary plant ingredient" should be understood to not encompass terpenoids, such as carotenoids (including β-carotenes etc.) and vitamin E, vitamin C, or their derivatives, as these compounds according to the present invention are defined to fall under the group of further antioxidants described in more detail herein below. Also the zinc and selenium compounds should fall under the definition of antioxidants rather than of phytochemicals.

The phytochemicals according to the invention can be derived from any plant, algae or fungus or combinations thereof but also comprise synthesized phytochemicals or combinations thereof as well as combinations of naturally occurring phytochemicals and synthesized phytochemicals.

The phytochemicals comprised by the compositions according to the present invention preferably fulfil an ORAC value of 5,000 to 20,000 µmol/g. The determination of the ORAC value is in detailed described in CAO, et al. Automated assay of oxygen radical absorbance capacity with the COBAS FARA II. Clinical Chemistry. 1995, vol.41, no.12. and provides a test for measuring the total anti-oxidative capacity of a substance or a nutrient. In a particularly preferred embodiment the ORAC value is within a range of from 7,000 to 15,000 µmol/g and more preferably within a range of from 10,000 to 15,000 µmol/g. Suitable phytochemicals providing an anti-oxidative capacity are known to the skilled person.

In a preferred embodiment a combination of phytochemicals that is derived from a natural source such as one or more plants algae and/or fungus is used as it has surprisingly been found that such combinations providing a broader spectrum of phytochemicals act in a synergistic manner. More preferably, combinations of phytochemicals derived from camomile, sage, arnica, rosemary, red wine and/or olive are comprised by the compositions of the present invention. The phytochemicals may be synthesized or extracted from natural sources by any suitable method known to those skilled in the art, particularly using food grade solvents. Liquid and solid, e.g. granulates or powder, extracts are suitable. Preferably the phytochemicals are employed according to the present invention as they occur in the respective natural source.

The term "phytochemical" is to be differentiated from the term "phytochemical compound". While a "phytochemical" is a composition that comprises additional substances, such as plant material, water, etc., a "phytochemical compound" refers to a single compound acting as an antioxidant and/or acting anti-inflammatory that is comprised by the "phytochemical", such as a secondary plant ingredient.

In particular embodiments the phytochemical is derived from plants, algae and/or fungus, wherein the term "derived from" also comprises synthesized phytochemical compounds that resemble single phytochemical compounds or combinations of phytochemical compounds as naturally found in plants, algae and/or fungus.

In preferred embodiments the phytochemical is derived from the whole plant or parts thereof, such as the flowers, the fruits the leaves and/or the roots.

The phytochemical may be contained as occurred within its natural source. The phytochemical may be present as concentrate, extract, such as alcoholic extract, lipophilic extract, distillate and/or aqueous extract, oil, expressed juices, or ground or otherwise minced natural material such as powders or combinations thereof.

Especially preferred embodiments of the phytochemical according to the invention comprise Chamomilla Recutita Extract (CAS no 84082-60-0), Chamomilla Recutita Oil (CAS no 8002-66-2), and camomile or sage distillate, extract, concentrate or oil.

In a further particular embodiment of the invention the phytochemical comprises one or more phytochemical compounds selected from the group consisting of monophenolic compounds; polyphenolic compounds including flavonoids, phenolic acids and non-flavonoids; glucosinolates; thiosulfonates; flavonoids such as anthocyanins, apigenin, catechines, isoflavones, hespertin, campherol, narginin, rutin, quercetin, silymarin, tangeretin, tannins, punicalagin, and luteolin; phenolic acids such as ellagic acid, chlorogenic acid, coumaric acid, phytic acid, ferulic acid, vanillin, cinnamic acid and hydrocinnamic acids; curcumin, resveratrol, lignans, (-)-α-bisabolol, bisabololoxide A, bisabololoxide B, En-In-dicycloether, matricin - also known as prochamazulen, apigenin-7-glucosid,thujon, α-thujon, β-thujon, 1,8-cineol, borneol, bornylester, sesquiterpens such as viridiflorol, β-caryophyllen and epoxidihydrocaryophyllen, and/or diterpens such as carnosol, rosmanol, safficinolid and salvin.

Preferably the phytochemical comprises a combination of at least three components selected from the group consisting of (-)-α-bisabolol, bisabololoxide A, bisabololoxide B, En-In-dicycloether, matricin, apigenin, and/or apigenin-7-glucosid. Bisabolol has been reported to have an antioxidant/anti-inflammatory activity ( BRAGA, PC, et al. Antioxidant activity of bisabolol: inhibitory effects on chemiluminescence of human neutrophil bursts and cell-free systems.. Pharmacology. 2008 Dec 18, vol.83, no.2, p.110-5. ).

In another preferred embodiment the phytochemical comprises a combination of at least three components selected from the group consisting of thujon, α-thujon, β-thujon, 1,8-cineol, borneol, bornylester, sesquiterpens, apigenin and/or luteolin.

Most preferably the phytochemical comprises apigenin, apigenin-7-glucosid, (-)-α-bisabolol and/or bisabololoxide A and/or B.

The composition of the invention comprises from 0.1 to 10 g, preferably from 0.2 to 8 g, and more preferably from 0.5 to 3 g of the phytochemical. In further preferred embodiments the amount of the phytochemical comprised by the composition of the invention is ≥ 0.3 g and ≤ 5 g, ≥ 0.5 g and ≤ 4 g, ≥ 1 g and ≤ 3 g. The amount of the phytochemical will depend on the nature of the phytochemical as well as the form in which it is present. For example, Chamomilla Recutita Oil will need to be present in a smaller amount than dried and minced powder derived from the camomile plant in order to achieve the same effect.

In one embodiment of the invention the amount of the phytochemical stated in the preceding paragraph is based on 100 kcal of the total composition.

In another embodiment of the invention the amounts of the phytochochemical given above are comprised by a single dose of the composition of the invention to be administered to a patient.

The nutritional compositions according to the present invention preferably further comprise an antioxidant or mixtures thereof as a third component.

An especially preferred antioxidant which additionally improves the anti-mucositis effect in accordance with the invention is the trace element zinc as the addition of zinc to the composition of the invention has surprisingly further improved the overall anti-mucositis effect, i.e. has shown to be beneficial in decreasing the severity of particularly chemo- and radiotherapy induced mucositis and oral discomfort.

The composition according to the invention preferably comprises 0.1 to 100 mg, preferably 1 to 75 mg, and more preferably 10 to 50 mg of zinc.

Further optional antioxidants may be comprised by the composition according to the invention as these surprisingly aid in the prevention or treatment of mucositis. Preferably, such an antioxidant is selected from the group consisting of terpenoids, vitamin E (comprising tocopherols and tocotrienols, both in α-, β-, γ and/or δ-forms), vitamin C, and selenium compounds as well as a mixture thereof. The terpenoids comprise carotenoid terpenoids and non-carotenoid terpenoids. The carotenoid terpenoids (mixed carotenoids) comprising α-carotene, β-carotene, γ-carotene, lutein, lycopene, and/or zeaxanthine are particularly preferable. Typical non-carotenoid terpenoids are the saponins, terpeneol, and terpene limonoids. The above mentioned compounds may be included into the nutritional compositions of the invention to generally combat oxidative stress and resultant genetic damage and slow the deterioration of collagen tissues.

In a further especially preferred embodiment the composition according to the invention furthermore comprises the antioxidant selenium as it was found that this micronutrient further improved the prophylaxis and treatment of mucositis.

In an even more preferred embodiment of the invention the composition at least comprises a combination of zinc and selenium as antioxidants. The combination of glutamine, a phytochemical, zinc and selenium has been found to be extremely effective in the treatment and/or prevention of mucositis.

In a further particularly preferred embodiment the composition comprises 0.25 to 10 mg and preferably 1 to 10 mg (mixed) carotenoids. More particular, the composition preferably comprises 0.1 to 10 mg and more preferably 0.5 to 5 mg β-carotene. Generally in 5 mg mixed carotenoids (cf. Table 1 below) about 2 mg β-carotene, 1.35 mg α-carotene, 0.65 mg lutein, 1 mg lycopene, and 0.1 mg zeaxanthine and γ-carotene are contained. In a further preferred embodiment the composition additionally comprises 3 to 300 mg, preferably 10 to 150 mg vitamin E, 10 to 500 mg, preferably 15 to 500 mg vitamin C; 3 to 300 µg, preferably 15 to 150 µg selenium; and/or 0.1 to 100 mg, preferably 1 to 75 mg zinc.

In one embodiment of the invention the amount of antioxidants stated in the preceding paragraphs is based on 100 kcal of the total composition.

In another embodiment of the invention the amounts of antioxidants given above are comprised by a single dose of the composition of the invention to be administered to a patient.

As further optional components the nutritional composition may comprise nutritional compounds commonly employed, such as stabilizers, colorants, flavors, pH adjusting agents, further vitamins (such as vitamin A, D, K, folic acid, thiamine, riboflavin, vitamin B6, vitamin B12, niacin etc.) minerals, sweeteners, anti-foam agents and fillers. The compositions may also additionally comprise macronutrients, such as fats, carbohydrates, proteins, and fiber components.

The nutritional composition according to the invention is for use as a medicament, preferably as a nutritional supplement, for the treatment and/or prevention of mucositis. It is particularly useful in the prevention of mucositis at a very early preclinical stage. It has surprisingly been found by the present inventors that there is an existing synergistic effect between the components of the composition and in particular between glutamine and the phytochemicals. Moreover, the additional presence of anti-oxidative vitamins and trace elements - especially selenium and/or zinc - further improves the anti-mucositis effects and leads to an increase of these effects being distinctly above the level to be expected from the sum of effects achieved by the respective single components alone. The present invention has been accomplished on the basis of such an unexpected finding. In view thereof, it has become possible according to the present invention for the first time to combine a treatment adapted to the underlying pathopyhsiology of mucositis in order to prevent the onset of mucositis by itself. The components of the composition act on the different sites of mucositis' onset.

Therefore, the use of the synergistic acting pharmaconutrients according to the invention renders it possible to counteract mucositis inducing metabolic changes. Without being bound by any theory, it is assumed that this synergistic effect is based on the action and positive influence of the compounds of the inventive composition at different sites of the inflammatory process in the mucosa.

The compositions according to the present invention are for use as a medicament, preferably as a nutritional supplement and/or a mouthwash, to be employed in the prevention and/or treatment of mucositis even in a pre-clinical stage of the disease that means already for preventing the onset of said disease but also the progression thereof. Furthermore, they are also suitable to be employed in the treatment of existing mucositis.

The medicament for the treatment and/or prevention of mucositis may furthermore comprise a pharmaceutically acceptable excipient.

The WHO scale comprises 4 grades of mucositis which are defined as follows: Grade I (mild): Oral soreness, erythema, Grade II (moderate): Oral erythema, ulcers solid diet tolerated, Grade III (severe): Oral ulcers, liquid diet only, Grade IV (life-threatening): Oral alimentation impossible.

The compositions according to the invention can be used for the treatment and/or the prevention of any of these four grades of mucositis. In preferred embodiments the compositions of the invention can be used for the treatment of grades III and/or IV as the compositions of the present invention will lead to an improvement of the condition of the patient and will thus allow the patient to return to a "normal" diet.

Particularly preferred embodiments of the composition according to the invention are in a form which is adapted to remain in the oral cavity for at least several seconds. In addition, the composition may be applicable for suckling or wetting of the oral cavity during chemo- and/or radiotherapy. Examples of such embodiments are, without limiting the scope of the invention, a drinkable mouth wash, a frozen aqueous solution, a viscous aqueous solution preferably cooled, a lozenge, an effervescent powder and a yoghurt-like composition.

The nutritional composition usually is present in a very low volume, having a viscous, and gel consistency. The product should have a composition which could be frozen, and sucked. The product should have frigorific properties, lasting at least 3-5 minutes on the oral mucosa/cavity, does not contain aggressive substances as e.g. alcohol, spicy or acid (like in fruit juices) and is not square-egged as e.g. hard candies. The patient should get the impression to have a supportive product as this could be sweeties. Suitable gel forming or gelling agents and cooling agents are those commonly used and known to the skilled person. The composition of the invention may contain various substances conventionally used as gelling agents or thickening agents in the field of food products. Examples of gelling agents include agar, gellan gum, carrageenan, pectin, pre-gelatinized starch, modified starch and gelatin. Examples of thickening agents include furcellaran, locust bean gum, guar gum, gum Arabic and xanthan gum. These gelling agents and thickening agents can be used singly or in combination. The combination of a gelling agent and a thickening agent is especially preferable. Gelling agents and/or thickening agents exhibit an appropriate gelling ability and gel stabilizing ability and control the gel strength of the resulting gel. When used in combination with agar, they can also mitigate water release and improve the texture of the resulting gel. The mixing operation of the components may be conducted at room temperature, but preferably with heating.

Possible product presentations are soft candy structure, a liquid solution which is frozen to "water ice", powder which is reconstituted with liquid to "wobble pudding" or jelly, or a ready-to-use jelly which could be cooled or frozen.

For the administration the respective components are either present in a single composition or in separate vehicles. The composition is preferably administered orally. However, also the enteral and intravenous administration is possible. The volume of the solution of the composition to be administered can be easily set to a reasonable value and usually is within a range of from 25 to 200 ml per serving. It is particularly suitable to administer the composition several times per day, most preferably twice or three times a day and during and/or shortly before the radio-/chemotherapy cycle.

The nutritional composition may be prepared by using methods known to the skilled person. In a preferred embodiment the respective components are simply mixed with each other in any order. However, it is also possible to encapsulate the single substances by known and commonly used encapsulation processes before mixing them. The encapsulation is usually done in order to improve the storage stability of the composition in dry form.

According to the invention, the composition is intended to comprise nutritional supplement compositions (nutritional supplements) and complete foodstuff compositions. In a preferred embodiment, the compositions according to the invention are a nutritional supplement, which is administered to the patient in a single dose once, twice or three times per day up to five times per day. Preferably, two or three single doses are administered per day. Even more preferably, at least one single dose is administered shortly before and/or during radio- or chemotherapeutical treatment of the patient. As described above, the term "single dose" refers to the amount of composition according to the invention that is administered to the patient in a continuous manner, i.e. without larger interruptions. Thus, a "single dose" may refer to one dose that is administered to the patient or it may refer to two, three, four or more sub-doses that are administered to the patient without larger interruptions of more than 10, 20, 30 or 60 minutes between two consecutive sub-doses. As an example, a "single dose" may consist of two or three sub-doses which are consecutively administered to the patient shortly before and during radiotherapy. If more than one single dose is administered per day the single doses can be of identical or different content of glutamine, phytochemical and/or antioxidant, respectively.

In further embodiments of the invention a daily dose of the composition comprises from 0.5 to 100 g, preferably from 5 g to 50 g, more preferably from 10 to 40 g, and most preferably from 15 to 30 g glutamine.

In further embodiments a daily dose of the inventive composition comprises 0.2 to 20 g, preferably from 0.4 to 16 g and more preferably from 1 to 6 g of phytochemicals per day.

In further embodiments a daily dose comprises 0.2 to 200 mg, preferably 5 to 150 mg and in particular 20 to 30 mg of zinc that are administered to a patient in order to further improve the anti-mucositis activity of the nutritional composition of the invention.

In further embodiments, a daily dose comprises 0.5 to 40 mg, preferably 10 to 40 mg, and more preferably 2 to 20 mg (mixed) carotenoids; 6 to 600 mg, preferably 20 to 300 mg vitamin E; 20 to 2000 mg, preferably 200 to 1000 mg vitamin C; and 6 to 750 µg, preferably 50 to 300 µg selenium are preferably administered to a patient in order to further improve the anti-mucositis activity of the nutritional composition of the invention.

Preferably, the compositions of the invention are administered orally.

In another embodiment of the invention it is particularly preferable to limit the caloric value of the composition to a specific value in order to minimize the impact of the supplement on the total diet of the patient. In other words, as the composition according to the present invention is intended to provide for a preventive effect on mucositis, and therefore is administered to persons not showing any nutritional deficiency symptom, the caloric value is kept as low as possible in some embodiments of the invention and then should not exceed 400 kcal per daily dose, preferably not more than 300 kcal per daily dose, and more preferably not more than 200 kcal per daily dose. In further specific embodiments of the invention, the caloric value should also not exceed 400 kcal per daily dose, preferably not more than 300 kcal per daily dose, and more preferably not more than 200 kcal per daily dose in case the composition according to the invention is used as a nutritional supplement in the treatment of mucositis or when the composition of the present invention is supplemented to other enteral or parenteral nutritional compositions. Generally, it is important to note that the amounts of glutamine and the phytochemical are remarkably high when taking the desired low total caloric value of the composition into account.

### Examples

### Example 1

A nutritional composition according to the invention is prepared by mixing the following components in the respective amounts in Table 1. No encapsulation is carried out prior to the mixing. The composition as indicated in Table 1 provides a caloric value of about 100 kcal. The amounts of the respective component correspond to 50% of the recommended daily dose so that the indicated composition should be administered twice a day. The caloric value can be determined by a suitable method known in the art.

**Table 1**

| **Component** | **Amount** |
|---|---|
| Glutamine | 15 g |
| Vitamin C | 250 mg |
| Vitamin E | 50 mg |
| Mixed carotenoids | 5 mg |
| Zinc | 15 mg |
| Selenium | 50 µg |
| Camomille or sage | 1.5 g |

The above mentioned composition is packed into sachets providing for a caloric value of about 100 kcal.

### Example 2

A mouth rinsing solution according to the invention was prepared according to Table 2 below. The solution is to be used at least twice a day to prevent and/or treat mucositis.

The solution was freshly prepared by diluting the powder mixture into 150 - 200 ml of water at room temperature.

**Table 2**

| **Component** | **Concentration/serving** |
|---|---|
| Glutamine | 15 g |
| Vitamin C | 250 mg |
| Vitamine E | 50 mg |
| Mixed carotinoids | 5 mg |
| Zinc | 15 mg |
| Selenium | 50 µg |
| German Camomile | 1.5 g |

### Example 3

A water based ice (50 ml/portion) according to the invention was prepared according to Table 3 below. The ice is preferably administered 4 times per day (daily dosage 4 servings/single doses).

The powder mixture (4 servings) was diluted in 200 ml water, distributed into 4 portions and frozen at -20°C in the freezer. The ice cubes can be suckled during the day and during and/or shortly before radio-/chemotherapy to prevent and/or treat mucositis.

**Table 3**

| **Component** | **Concentration/serving** |
|---|---|
| Glutamine | 7.5 g |
| Vitamin C | 125 mg |
| Vitamine E | 25 mg |
| Mixed carotinoids | 2.5 mg |
| Zinc | 7.5 mg |
| Selenium | 25 µg |
| Sage | 0.75 g |

### Example 4

A gel with cooling properties (50 g/serving) according to the invention was prepared according to Table 4 below. The cooling gel is preferably administered 4 times per day (daily dosage 4 servings/single doses).

The powder mixture (single serving) was diluted in 50 ml cold water. The gel can be suckled during the day and during and/or shortly before radio-/chemotherapy to prevent and/or treat mucositis.

**Table 4**

| **Component** | **Concentration/serving** |
|---|---|
| Glutamine | 7.5 g |
| Vitamin C | 125 mg |
| Vitamine E | 25 mg |
| Mixed carotinoids | 2.5 mg |
| Zinc | 75 mg |
| Selenium | 25 µg |
| Olive extract | 1 g |
| Thick & Easy | 4.5 g |

Thick & Easy consists of 100 % modified corn starch and is available from Fresenius Kabi.

### Example 5

A jelly according to the invention was prepared according to Table 5 below. The powder mixture (single serving) was diluted in 100 ml cold water; the powder forms a jelly consistency. The jelly can be consumed during the day as in-between meal to prevent and/or treat mucositis. The jelly is preferably administered in a daily dosage 3 of servings/single doses.

**Table 5**

| **Component** | **Concentration/serving** |
|---|---|
| Glutamine | 10 g |
| Vitamin C | 167 mg |
| Vitamine E | 33 mg |
| Mixed carotinoids | 3.3 mg |
| Zinc | 10 mg |
| Selenium | 33 µg |
| Red wine extract | 1 µg |
| Thick & Easy | 4.5 g |

Example 6

Examples 1-3 were repeated, wherein the camomille or sage powder, respectively was exchanged for camomille and sage extracts, respectively with similar results. The amount of extract added was 50 % of the amount of the powder added in examples 1-3.

## Claims

1. A nutritional composition comprising:
a) from 2.5 to 18 g of glutamine in neutral or salt form or a precursor or derivative; and
b) from 0.1 to 10 g of a phytochemical or a mixture thereof, wherein the phytochemical is an antioxidant.

2. The composition according to claim 1, wherein the phytochemical further has an anti-inflammatory effect.

3. The composition according to claim 1 or 2, wherein the phytochemical or mixture thereof provides an anti-oxidative capacity with an ORAC value of from 5,000 to 20,000 µmol/g, preferably in a range of 7,000 to 15,000 µmol/g, more preferably in a range of 10,000 to 15,000 µmol/g.

4. The composition according to anyone of the preceding claims, wherein the phytochemical is derived from camomile, sage, arnica, rosemary, red wine and/or olive.

5. The composition according to any on of the preceding claims, wherein the phytochemical comprises one or more phytochemical compounds selected from the group consisting of monophenolic compounds, polyphenolic compounds, glucosinolates, thiosulfonates, (-)-α-bisabolol, bisabololoxide A, bisabololoxide B, En-In-dicycloether, matricin, apigenin, apigenin-7-glucosid,thujon, α-thujon, β-thujon, 1,8-cineol, borneol, bornylester, luteolin, sesquiterpens and/or diterpens.

6. The composition according to any of the preceding claims, further comprising:
c) one or more antioxidants selected from the group consisting of carotenoids, vitamin E, vitamin C, zinc and selenium.

7. The composition according to any one of the preceding claims, comprising
a) from 2.5 to 18 g of glutamine in neutral or salt form or a precursor or derivative thereof,
b) from 0.1 to 10 g of a phytochemical or a mixture thereof,
c) from 0.25 to 10 mg mixed carotenoids, 3 to 300 mg vitamin E, 10 to 500 mg vitamin C, 3 to 300 µg selenium and 0.1 to 100 mg of zinc.

8. The composition according to any of the preceding claims, wherein the amount of components a), b) and/or c) is based on 100 kcal of the total composition.

9. The composition according to any of the preceding claims, wherein the amount of components a), b) and/or c) is comprised by a single dose of the composition to be administered to a patient.

10. The composition according to any one of the preceding claims, which additionally comprises a gel forming agent and/or a cooling agent.

11. The composition according to any one of claims 1-10 as a medicament, preferably as a nutritional supplement and/or a mouth-wash.

12. The composition according to any one of claims 1-11 for the prevention and/or treatment of mucositis, preferably in chemo- and radiotherapy.

13. The composition according to any of the preceding claims, wherein the composition is to be administered in a daily dose comprising:
(a) from 2.5 to 100 g of a glutamine,
(b) from 0.2 to 20 g of a phytochemical,
(c) optionally 0.5 to 40 mg mixed carotenoids, 6 to 600 mg vitamin E, 20 to 2000 mg vitamin C, 6 to 750 µg selenium and 0.2 to 200 mg zinc.

14. The composition according to any one of claims 1-12, which is administered to the patient orally one to five times per day, preferably twice or three times per day.

15. Sachet comprising the composition according to any one of claims 1-9, which provides for a caloric value of not more than 200 kcal, preferably not more than 150 kcal and more preferably not more than 100 kcal.
